# FASCICULE DE BREVET EUROPEEN

(11) **EP 4 056 154 B1**
(45) Date de publication et mention de la délivrance du brevet: **13.08.2025**
(21) Numéro de dépôt: 22158729.8
(22) Date de dépôt: 25.02.2022
(51) Int. Cl.: A61F 2/40, A61F 2/46

(54) **IMPLANT D ESSAI GLÉNOÏDIEN POUR PROTHÈSE TOTALE D ÉPAULE INVERSÉE**
SCHULTERGELENKPFANNEN-PROBEIMPLANTAT FÜR EINE INVERSE SCHULTER-TOTALPROTHESE
GLENOID TRIAL IMPLANT FOR A TOTAL REVERSE SHOULDER PROSTHESIS

(30) Priorité: 12.03.2021 FR 2102495
(43) Date de publication de la demande: 14.09.2022
(73) Titulaire: FX Shoulder Solutions, 01440 Viria (FR)
(72) Inventeur: DAUDET, Jean-Marie, 01000 BOURG-EN-BRESSE (FR)
(74) Mandataire: LLR

(56) Documents cités:
- WO-A1-01/47442
- WO-A1-2016/160417

## Description

L'invention concerne un implant d'essai glénoïdien de prothèse totale de l'épaule inversée, destiné à être fixé temporairement à la cavité glénoïdienne du patient. L'état de la technique le plus proche est le document WO 2016/160417 A1, qui définit le préambule de la revendication 1.

Une prothèse totale de l'épaule inversée comprend un implant huméral, c'est-à-dire une partie fixée dans l'humérus du patient, et un implant glénoïdien, c'est-à-dire une partie fixée sur la cavité glénoïdienne (aussi appelée glène) du patient. Ces deux implants sont articulés l'un avec l'autre afin de restaurer la mobilité du bras du patient opérée.

Dans le cadre d'une prothèse totale de l'épaule dite « inversée », le centre de rotation de l'articulation est déplacé sur l'implant glénoïdien, à l'inverse d'une prothèse anatomique où il demeure situé sur l'implant huméral.

Cette modification du positionnement du centre de rotation de l'articulation engendre une modification des muscles sollicités pour mouvoir le bras. En l'espèce, c'est le muscle deltoïde qui est sollicité tandis que pour une prothèse anatomique, les muscles sollicités demeurent ceux formant la coiffe des rotateurs, à savoir les muscles : sous-scapulaire, sus-épineux, sous-épineux et petit rond en plus du deltoïde. Cette modification des muscles impliqués peut s'expliquer par un endommagement irréparable de tout ou partie des muscles de la coiffe des rotateurs, un tel endommagement pouvant intervenir chez les personnes âgées notamment.

La prothèse d'épaule inversée implique donc la sollicitation du muscle deltoïde qui est ancré sur l'humérus de manière plus distale que les muscles de la coiffe des rotateurs. Par conséquent, il est nécessaire pour que le muscle deltoïde soit fonctionnel dès les premiers degrés d'abduction de déplacer le centre de rotation de la prothèse sur la glène. De plus pour utiliser le deltoïde pour les rotations interne et externe, il est nécessaire de le tendre. Une telle tension du muscle deltoïde peut être réalisée en latéralisant e et/ou allongeant longitudinalement l'humérus. En d'autres termes, on latéralise et/ou on abaisse l'humérus par rapport à sa position naturelle afin d'augmenter les tensions qui s'appliquent sur le muscle deltoïde.

Ce déport latérale et/ou longitudinale du centre de rotation ainsi située sur l'implant glénoïdien peut être obtenu respectivement par une augmentation de l'épaisseur totale de l'implant (variabilité du diamètre de la glénosphère ou de l'épaisseur de la métaglène) ou par une décentration entre la glénosphère et la métaglène formant l'implant glénoïdien (il existe actuellement des implants glénoïdiens pour lesquels la glénosphère est décentrée par rapport à la métaglène permettant d'abaisser le centre de rotation plus ou moins en fonction du diamètre de la glénosphère).

En conséquence, le choix de l'implant glénoïdien est prépondérant au succès de la chirurgie et de la survie de la prothèse totale d'épaule dans le temps. Ces implants étant des composants stériles sous vide qu'il n'est pas possible de stériliser à nouveau, il est dès lors nécessaire de savoir quel implant utilisé avant même d'ouvrir l'emballage comprenant l'implant, au risque de déstériliser un implant inadapté pour le patient, cet implant devenant inutilisable.

Afin de choisir l'implant glénoïdien le plus adapté possible au patient, sans pour autant le sortir de son emballage stérile, il est connu de recourir à des logiciels permettant une planification en 3D lors des périodes préopératoire. Cependant, ces solutions présentent plusieurs inconvénients. En effet, elles nécessitent un équipement électronique sophistiqué et onéreux. En outre, elles impliquent un long travail préopératoire qui occupe le chirurgien, celui-ci n'étant pas disponible pour d'autres tâches durant cette période. Enfin, elles ne permettent pas de choisir avec certitude l'implant le plus adapté puisque les logiciels de planification ne permettent pas de prendre en compte toutes les variables impliquées dans l'articulation entre l'humérus et la glène, par exemple, la tension ligamentaire résultante de la combinaison choisie des implants huméral et glénoïdien et de leur mise en place.

L'invention a notamment pour but de permettre de définir avec certitude l'implant glénoïdien le plus adapté pour le patient de sorte à réduire les implants déstérilisés par erreur. L'invention vise aussi à permettre de raccourcir drastiquement le temps préopératoire du chirurgien. L'invention vise enfin à s'affranchir des solutions onéreuses telles que celles recourant à l'utilisation de logiciel de planification en proposant une solution bon marché.

A cet effet l'invention a pour objet un implant d'essai glénoïdien pour prothèse totale d'épaule inversée tel que défini dans la revendication 1.

Un implant d'essai glénoïdien correspond à un implant fixé par le chirurgien pendant la période opératoire, de manière provisoire, afin de réaliser des essais quant à l'emplacement définitif du centre de rotation de l'articulation de l'épaule. Dans le cadre des prothèses d'épaule inversées le centre de rotation de l'articulation de l'épaule correspondant à l'axe de révolution de la glénosphère. L'implant d'essai a vocation à être retiré, soit pour qu'un autre implant d'essai soit essayé par le chirurgien, soit, lorsque l'emplacement définitif du centre de rotation de l'articulation de l'épaule est identifié, être remplacé par l'implant définitif correspondant.

La métaglène de l'implant d'essai selon l'invention peut être de forme circulaire, ovoïde ou piriforme.

La glénosphère de l'implant d'essai selon l'invention peut être de forme hémisphérique ou être une glénosphère de plus d'une demie-sphère.

Ainsi, le chirurgien peut juger en direct de la mobilité possible de l'épaule grâce à la combinaison d'un implant d'essai glénoïdien selon l'invention et d'un implant d'essai huméral. En effet, le chirurgien peut tester la tension ligamentaire et musculaire résultante de la combinaison choisie et mise en place, ce qui lui permet de définir de manière certaine l'implant glénoïdien adapté pour l'opération en cours, et ce, de manière simple puisqu'il n'a pas besoin de recourir à l'utilisation de logiciel de planification ou de navigation.

Dès lors, l'implant définitif glénoïdien adapté pour l'opération en cours peut être retiré de son emballage stérile uniquement lorsque le chirurgien est certain qu'il s'agit de l'implant adéquat. On réduit donc, grâce à l'invention, de manière drastique l'ouverture par erreur d'implants définitifs glénoïdiens se révélant ensuite inappropriés pour l'opération en cours.

Suivant d'autres caractéristiques optionnelles de l'invention prises seules ou en combinaison :
- les axes de révolution de la métaglène et de la glénosphère sont confondus ;
- les axes de révolution de la métaglène et de la glénosphère sont sécants ;
- les axes de révolution de la métaglène et de la glénosphère sont strictement parallèles ;
- les axes de révolution de la métaglène et de la glénosphère sont espacées l'un de l'autre d'une distance comprise entre 0.5 et 5 mm, de préférence entre 1 et 3 mm ;
- la métaglène comprend une butée agencée pour former entretoise entre la glénosphère et la cavité glénoïde lorsque l'implant d'essai est assemblé au sein de la cavité glénoïde, ladite butée ayant une épaisseur inférieure ou égale à 10 mm, de préférence inférieure ou égale à 6 mm ;
- la métaglène et la glénosphère sont réalisées d'un seul tenant, formant un implant monobloc ;
- l'implant d'essai glénoïdien comprend des moyens d'assemblage la métaglène et la glénosphère ;
- la métaglène et/ou la glénosphère est réalisée en polymère plastique ou en acier inoxydable ou en titane et en un alliage de ces matériaux.

L'invention a également pour objet un ensemble de comprenant plusieurs implants d'essai glénoïdiens selon l'une quelconque des variantes possibles.

Ainsi, il est possible de fournir au chirurgien un ensemble (pouvant revêtir la forme d'un kit ou d'une boite d'implants d'essai) comprenant une pluralité d'implants d'essai glénoïdiens selon l'invention. Cette pluralité d'implants d'essai glénoïdiens permet au chirurgien d'avoir à disposition toutes les combinaisons possibles lui permettant d'ajuster au mieux et pendant l'opération le centre de rotation de l'articulation de l'épaule en fonction des particularités morphologiques du patient.

L'invention a également pour objet un procédé de fixation d'une prothèse totale d'épaule inversée chez un patient comprenant les étapes de :
a) fixation d'un implant d'essai glénoïdien selon l'une quelconque des revendications 1 à 9 au sein de la cavité glénoïde du patient,
b) fixation d'un implant d'essai huméral au sein de l'humérus du patient,
c) vérification de la bonne compatibilité des implants d'essai glénoïdien et huméral en vue du positionnement du centre de rotation de l'articulation de l'épaule à l'emplacement adéquat en fonction des particularités morphologiques du patient.

Suivant d'autres caractéristiques optionnelles du procédé selon l'invention prises seules ou en combinaison :
- si la compatibilité est avérée entre les implants d'essai glénoïdien et huméral, on répète au moins l'une des étapes a), b) ou c), avec un implant d'essai glénoïdien et/ou un implant d'essai huméral de dimensions différentes ;
- si la compatibilité est avérée entre les implants d'essai glénoïdien et huméral, ces derniers sont retirés et remplacer par des implants définitifs glénoïdien et huméral de dimensions identiques.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est un ensemble de vues schématiques représentant les implants humoral et glénoïdien formant une prothèse totale d'épaule inversée ;
[Fig. 2] la figure 2 est un ensemble de vues schématiques représentant des implants glénoïdien selon plusieurs variantes de l'invention ;
[Fig. 3] la figure 3 est un ensemble de vues schématiques représentant des implants glénoïdien selon plusieurs variantes de l'invention ;
[Fig. 4] la figure 4 est un ensemble de vues schématiques représentant des implants glénoïdien selon plusieurs variantes de l'invention.

### Description détaillée

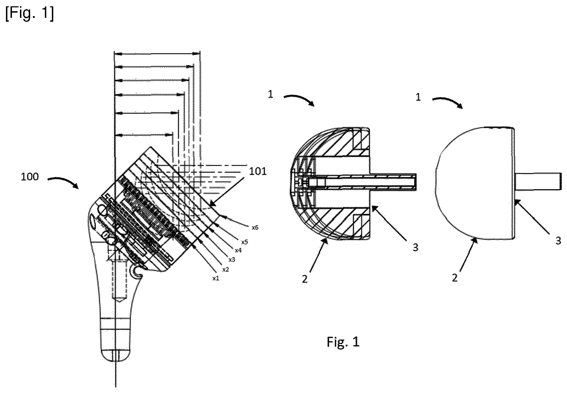

On a représenté sur les figures 1 à 4 des implants d'essai glénoïdien pour prothèse totale d'épaule inversée, désigné par la référence générale 1.

Comme représenté à la figure 1, l'implant d'essai glénoïdien 1 selon l'invention comprend une métaglène 3 présentant un axe de révolution d1 et étant destinée à être assemblée de manière stabilisée et provisoire au sein de la cavité glénoïde (non représentée) d'un patient à opérer. Dans les variantes représentées aux figures, la métaglène 3 est de forme circulaire. L'implant 1 comprend également une glénosphère 2 présentant un axe de révolution (d2) et étant assemblée solidairement à la métaglène 3 et destinée à être reçue au sein d'une cupule 101 d'un implant d'essai huméral 100. Dans les variantes représentées aux figures, la glénosphère 2 est de plus d'une demi-sphère.

Ainsi, le chirurgien dispose d'un implant d'essai 1 qu'il peut fixer à la cavité glénoïde en vue de définir, durant l'opération, la meilleure position pour le centre de rotation de l'articulation de l'épaule. Lorsqu'une telle position est atteinte, il peut choisir l'implant définitif glénoïdien dont la forme correspond à l'implant d'essai 1. Ainsi, l'implant définitif est sorti de son emballage stérile uniquement lorsque le chirurgien est certain qu'il correspond au bon implant à utiliser.

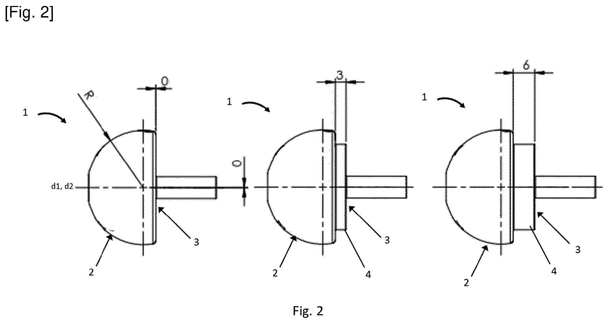

Selon des variantes de réalisation de l'invention, représentées à la figure 2, les axes de révolution d1 et d2 de la métaglène 3 et de la glénosphère 2 sont confondus, permettant ainsi de centrer ces deux éléments l'un par rapport à l'autre. Cette configuration est particulièrement avantageuse lorsque l'abaissement longitudinal du centre de rotation de l'articulation de l'épaule (qui correspond à l'axe de révolution d2 de la glénosphère 2) n'est pas nécessaire.

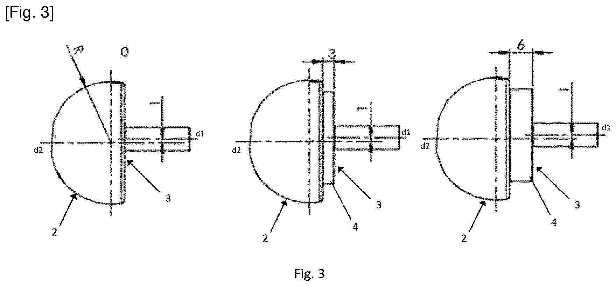

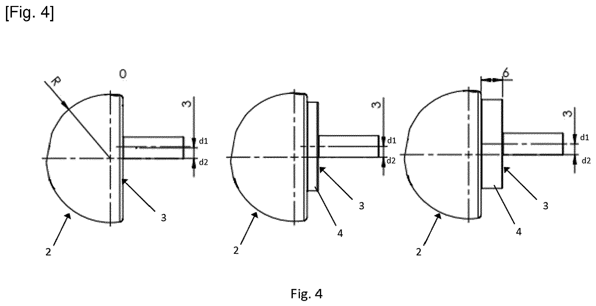

Alternativement, comme représenté aux figures 3 et 4, les axes de révolution d1 et d2 de la métaglène 3 et la glénosphère 2 sont strictement parallèles, ce qui signifie que ces deux éléments son décentrés l'un par rapport à l'autre. Selon les variantes de l'invention représentées à la figure 3, la métaglène 3 et la glénosphère 2 sont décentrées l'une de l'autre longitudinalement, par rapport au corps du patient, d'une distance de 1 mm, permettant *in fine* d'abaisser la position du centre de rotation de l'articulation de l'épaule, ce qui engendre également l'abaissement longitudinal de l'humérus. Cela permet de tendre le muscle deltoïde selon une direction longitudinale. Une telle décentration permet également de position la métaglène un peu ou plus ou un peu plus bas au regard des propriétés morphologiques du patient. Une telle variation peut également permettre de positionner le centre de rotation de l'articulation de l'épaule à la position la plus adéquate. Selon les variantes de l'invention représentées à la figure 4, la distance séparant les axes de révolution d1 et d2 est de 3 mm, permettant ainsi d'abaisser encore davantage le centre de rotation de l'articulation et donc, *in fine,* d'appliquer une tension encore plus forte sur le muscle deltoïde.

La métaglène 3 comprend une butée 4 (visible sur les figures 2 à 4) agencée pour former entretoise entre la glénosphère 2 et la cavité glénoïde lorsque l'implant d'essai 1 est assemblé au sein de cette dernière. Comme représenté, l'épaisseur de cette butée 4 varie jusqu'à 6 mm. Cette variation de l'épaisseur de la butée 4 permet une latéralisation du centre de rotation de l'articulation de l'épaule et, par voie de conséquence, une latéralisation de l'humérus une fois la prothèse d'épaule assemblée. Il en résulte l'application d'une tension plus forte sur le muscle deltoïde selon une direction latérale.

Ainsi, il est possible de tendre suffisamment le muscle deltoïde pour que celui-ci soit fonctionnel dès les premiers degrés d'abduction en remplacement des muscles formant la coiffe des rotateurs.

Enfin, il est également possible de faire varier le diamètre de la glénosphère 2 d'un implant d'essai glénoïdien 1 afin de déporter encore davantage son axe de révolution d2 (et donc de faire varier longitudinalement et/ou latéralement le centre de rotation de l'articulation de l'épaule). Le diamètre de telles glénosphères 2 peut varier entre 30 et 44 mm.

L'implant d'essai glénoïdien 1 peut être monobloc, ce qui signifie que la métaglène 3 et la glénosphère 2 sont réalisées d'un seul tenant et ne peuvent pas être séparées l'une de l'autre.

Alternativement, l'implant 1 comprend des moyens d'assemblage permettant de fixer solidairement la métaglène 3 et la glénosphère 2 de sorte qu'elles ne se désolidarisent pas lorsque l'implant 1 est fixé au sein de la cavité glénoïde ou lorsque que le chirurgien réalise des tests durant l'opération. De tels moyens d'assemblage sont connus de l'homme du métier. A titre d'exemples non limitatifs, il peut s'agir d'organes de clipsage, d'une fixation vis/écrou, *etc.*

Un ensemble de plusieurs d'implants d'essai glénoïdiens 1 selon des variantes différentes de l'invention peut être mis à disposition du chirurgien sous la forme d'un kit d'essai. Ainsi, le chirurgien dispose de toutes les variantes possibles d'implants d'essai glénoïdiens 1 lui permettant d'ajuster au mieux le centre de rotation de l'articulation de l'épaule, en fonction des particularités morphologiques du patient. Une fois la bonne position du centre de rotation de l'articulation atteinte, il ne lui reste plus qu'à choisir l'implant glénoïdien définitif de forme identique à l'implant d'essai glénoïdien 1 avec lequel ladite position a été atteinte.

L'invention n'est pas limitée aux modes de réalisation présentés et d'autres modes de réalisation apparaîtront clairement à l'homme du métier. Il est notamment possible d'utiliser des glénosphère possédant des diamètres différents de ceux cités ou de décentrer encore davantage la métaglène par rapport à la glénosphère.

### Liste de références

1 : implant d'essai glénoïdien
2 : glénosphère
3 : métaglène
4 : butée
100 : implant d'essai humoral
101 : cupule
d1 : axe de révolution de la métaglène
d2 : axe de révolution de la glénosphère

## Revendications

1. Implant d'essai glénoïdien (1) pour prothèse totale d'épaule inversée, comprenant une glénosphère (2) présentant un axe de révolution (d2) et destinée à être reçue au sein d'une cupule (101) d'un implant d'essai huméral (100), **caractérisé en ce que** l'implant d'essai glénoïdien comprend en outre une métaglène (3) présentant un axe de révolution (d1) et étant destinée à être assemblée de manière stabilisée et provisoire au sein de la cavité glénoïde d'un patient à opérer, la glénosphère étant adaptée à être assemblée solidairement à la métaglène.

2. Implant d'essai glénoïdien (1) selon la revendication 1, dans lequel les axes de révolution (d1, d2) de la métaglène (3) et de la glénosphère (2) sont confondus.

3. Implant d'essai glénoïdien (1) selon la revendication 1, dans lequel les axes de révolution (d1, d2) de la métaglène (3) et de la glénosphère (2) sont sécants.

4. Implant d'essai glénoïdien (1) selon la revendication 1, dans lequel les axes de révolution (d1, d2) de la métaglène (3) et de la glénosphère (2) sont strictement parallèles.

5. Implant d'essai glénoïdien (1) selon la revendication précédente, dans lequel les axes de révolution (d1, d2) de la métaglène (2) et de la glénosphère (3) sont espacées l'un de l'autre d'une distance comprise entre 0.5 et 5 mm, de préférence entre 1 et 3 mm.

6. Implant d'essai glénoïdien (1) selon l'une quelconque des revendications précédentes, dans lequel la métaglène (3) comprend une butée (4) agencée pour former entretoise entre la glénosphère (2) et la cavité glénoïde lorsque l'implant d'essai (1) est assemblé au sein de la cavité glénoïde, ladite butée (4) ayant une épaisseur inférieure ou égale à 10 mm, de préférence inférieure ou égale à 6 mm.

7. Implant d'essai glénoïdien (1) selon l'une quelconque des revendications précédentes, dans lequel la métaglène (3) et la glénosphère (2) sont réalisées d'un seul tenant, formant un implant (1) monobloc.

8. Implant d'essai glénoïdien (1) selon l'une quelconque des revendications 1 à 6, comprenant des moyens d'assemblage la métaglène (3) et la glénosphère (2).

9. Implant d'essai glénoïdien (1) selon l'une quelconque des revendications précédentes, dans lequel la métaglène (3) et/ou la glénosphère (2) est réalisée en polymère plastique ou en acier inoxydable ou en titane et en un alliage de ces matériaux.

10. Ensemble comprenant plusieurs implants d'essai glénoïdiens (1) selon l'une quelconque des revendications précédentes.

## Patentansprüche

1. Schultergelenkpfannen-Probeimplantat (1) für eine inverse Schultertotalprothese, umfassend eine Glenosphäre (2), die eine Rotationsachse (d2) aufweist und dazu bestimmt ist, in einer Pfanne (101) eines humeralen Probeimplantats (100) aufgenommen zu werden,
**dadurch gekennzeichnet, dass** das Schultergelenkpfannen-Probeimplantat ferner eine Basisplatte (3) umfasst, die eine Rotationsachse (d1) aufweist und dazu bestimmt ist, stabilisierend und provisorisch in der Schultergelenkpfanne eines zu operierenden Patienten zusammengebaut zu werden, wobei die Glenosphäre eingerichtet ist, um mit der Basisplatte fest zusammengebaut zu werden.

2. Schultergelenkpfannen-Probeimplantat (1) nach Anspruch 1, wobei die Rotationsachsen (d1, d2) der Basisplatte (3) und der Glenosphäre (2) zusammenfallen.

3. Schultergelenkpfannen-Probeimplantat (1) nach Anspruch 1, wobei die Rotationsachsen (d1, d2) der Basisplatte (3) und der Glenosphäre (2) sich schneiden.

4. Schultergelenkpfannen-Probeimplantat (1) nach Anspruch 1, wobei die Rotationsachsen (d1, d2) der Basisplatte (3) und der Glenosphäre (2) genau parallel sind.

5. Schultergelenkpfannen-Probeimplantat (1) nach dem vorhergehenden Anspruch, wobei die Rotationsachsen (d1, d2) der Basisplatte (2) und der Glenosphäre (3) um einen Abstand zwischen 0,5 und 5 mm, bevorzugt zwischen 1 und 3 mm, voneinander beabstandet sind.

6. Schultergelenkpfannen-Probeimplantat (1) nach einem der vorhergehenden Ansprüche, wobei die Basisplatte (3) einen Anschlag (4) umfasst, der angeordnet ist, um einen Abstandshalter zwischen der Glenosphäre (2) und der Schultergelenkpfanne zu bilden, wenn das Probeimplantat (1) in der Schultergelenkpfanne zusammengebaut wird, wobei der Anschlag (4) eine Dicke hat, die kleiner oder gleich 10 mm, bevorzugt kleiner oder gleich 6 mm ist.

7. Schultergelenkpfannen-Probeimplantat (1) nach einem der vorhergehenden Ansprüche, wobei die Basisplatte (3) und die Glenosphäre (2) einstückig realisiert sind und ein Monoblock-Implantat (1) bilden.

8. Schultergelenkpfannen-Probeimplantat (1) nach einem der Ansprüche 1 bis 6, umfassend Mittel zum Zusammenbauen der Basisplatte (3) und der Glenosphäre (2).

9. Schultergelenkpfannen-Probeimplantat (1) nach einem der vorhergehenden Ansprüche, wobei die Basisplatte (3) und/oder die Glenosphäre (2) Kunststoffpolymer oder aus rostfreiem Stahl oder aus Titan oder aus einer Legierung aus diesen Materialien ist.

10. Anordnung, umfassend mehrere Schultergelenkpfannen-Probeimplantate (1) nach einem der vorhergehenden Ansprüche.

## Claims

1. Glenoid trial implant (1) for reverse total shoulder prosthesis, comprising a glenosphere (2) having an axis of revolution (d2) and intended to be received inside a cupula (101) of a humeral trial implant (100),
**characterized in that** the glenoid trial implant (1) comprises further a metaglene (3) having an axis of revolution (d1) and being intended to be assembled in a stabilized and temporary manner inside the glenoid fossa of a patient who will receive surgery, and the glenosphere (2) being integrally assembled to the metaglene.

2. Glenoid trial implant (1) according to claim 1, wherein the axes of revolution (d1, d2) of the metaglene (3) and of the glenosphere (2) coincide.

3. Glenoid trial implant (1) according to claim 1, wherein the axes of revolution (d1, d2) of the metaglene (3) and of the glenosphere (2) intersect.

4. Glenoid trial implant (1) according to claim 1, wherein the axes of revolution (d1, d2) of the metaglene (3) and of the glenosphere (2) are strictly parallel.

5. Glenoid trial implant (1) according to the preceding claim, wherein the axes of revolution (d1, d2) of the metaglene (2) and of the glenosphere (3) are separated from each other by a distance of between 0.5 and 5 mm, preferably between 1 and 3 mm.

6. Glenoid trial implant (1) according to any one of the preceding claim, wherein the metaglene (3) comprises a stop (4) designed to act as a spacer between the glenosphere (2) and the glenoid fossa when the trial implant (1) is assembled inside the glenoid fossa, the thickness of said stop (4) being less than or equal to 10 mm, preferably less than or equal to 6 mm.

7. Glenoid trial implant (1) according to any one of the preceding claim, wherein the metaglene (3) and the glenosphere (2) are made in one piece, forming a monoblock implant (1).

8. Glenoid trial implant (1) according to any one of claims 1 to 6, comprising means for assembling the metaglene (3) and the glenosphere (2).

9. Glenoid trial implant (1) according to any one of the preceding claim, wherein the metaglene (3) and/or the glenosphere (2) are made from plastic polymer or stainless steel or titanium and an alloy of these materials.

10. Set comprising several glenoid trial implants (1) according to any one of the previous claims.
